# EUROPEAN PATENT APPLICATION

(11) **EP 2 335 499 A1**
(43) Date of publication of application: **22.06.2011**
(21) Application number: 09178491.8
(22) Date of filing: 09.12.2009
(51) Int. Cl.: A23L 1/164, A23L 1/09, A23L 1/105, A23L 1/308

(54) **Process for making a whole-grain cereal bar and cereal bar**

(71) Applicant: Nestec S.A., 1800 Vevey (CH)
(72) Inventor: Roger, Olivier, 1510, MOUDON (CH); Bousquet, Aude, 1030, BUSSIGNY (CH); Wavreille, Anne-Sophie, 1004, Lausanne (CH); Chisholm, Helen, 1033, Cheseaux (CH); Mathys, Loïc, 51520 Sarry (CH); Schaffer-Lequart, Christelle, 1083, MEZIERES (CH); Schmidt, Isabelle, 1400 Yverdon-les-Bains (CH)
(74) Representative: Cogniat, Eric Jean Marie

(57) **Abstract**

A process for preparing a cereal bar comprising the steps of i) blending a food pieces mix comprising whole grain cereal pieces, with a whole grain cereal binder, and ii) forming a cereal bar from said mixture, wherein said binder is obtainable by a process comprising the steps of:
a) contacting a whole grain cereal flour in water with an enzyme composition comprising at least a protease and an alpha-amylase, both showing no hydrolytic activity towards dietary fibres, to provide a whole grain flour hydrolysate,
b) inactivating said enzymes when said hydrolysate has reached a stable viscosity comprised between 100 and 30,000 mPa.s measured at 65°C and a total solid content of 25 to 50% by weight,
c) concentrating and mixing with a humectant and a fat component, to provide a whole grain binder.

## Description

### TECHNICAL FIELD

The invention relates to a process for preparing a cereal bar with a high whole grain cereal content. The invention also relates to a cereal bar having a high whole grain cereal content, which is obtainable by such a process. In particular, such cereal bars contain dietary fibres. The invention also relates to a process for preparing a whole grain cereal binder which may be used in the manufacture of such cereal bars.

### BACKGROUND OF THE INVENTION

There is now extensive evidence emerging mainly from epidemiological studies that a daily intake of three servings of whole grain products, i.e. 48 g of whole grain, is positively associated with decreased risk of cardiovascular diseases, increased insulin sensitivity and decreased risk of type 2 diabetes onset, obesity (mainly visceral obesity) and digestive system cancers. These health benefits are reported to be due to the synergistic role of fibre and other components, such as vitamins, minerals and bioactive phytochemicals.

The regulatory authorities in Sweden, the US and the UK have already approved specific heart health claims based on the available scientific substantiation.

Whole grain food products are also growing in popularity with consumers, not just because whole grain consumption is now included in some national dietary recommendations but also because whole grain products are considered as wholesome and natural. Recommendations for whole grains consumption have been set up by government authorities and experts groups to encourage consumers to eat whole grains. For instance, in the U.S.A, recommendations are to consume 3 to 5 servings per day, with 16 g of whole grain ingredient per serving. However, data provided by national dietary surveys in the United Kingdom, the U.S.A. and China show that whole grain consumption varies between 0 and 2 servings/day.

The lack of whole grain products offered on the shelves and the poor palatability of available ones are generally identified as barriers for whole grain consumption. Processes to increase whole grain content in available products with acceptable palatability can advantageously be used to increase the overall whole grain consumption.

Whole cereal grains are a recognised source of dietary fibres, phytonutrients, antioxidants, vitamins and minerals. According to the definition given by the American Association of Cereal Chemists (AACC), whole grains cereal, and food made from them, consist of the entire grain seed. The entire grain seed comprises the germ, the endosperm and the bran. It is usually referred to as the kernel.

Cereal bars, also called granola bars, are known in the art for providing whole grain cereal. For instance, European patent application EP 1782698 A1 discloses a low-calorie whole grain cereal bar which comprises whole grain, a binder and a compound coating. More specifically, the cereal bar provides at least 15 % by weight of whole grain, at least 5 % by weight of protein, at least 5 % by weight of fibre, with less than 120 calories per 28 gram bar. The binder provides 25 to 35 % by weight of the soluble fibre content of the cereal bar. In order to achieve this, soluble fibres are added as a powder to the syrup, when preparing the binder. Thus the fibres in this cereal bar are an additive in the binder. Therefore, such a product cannot be considered as a whole grain product, in which the source of fibres is only the whole grains used during manufacture of the cereal bar.

Furthermore this binder does not contain any whole grain. Examples of bars, containing between 11.4 % by weight and 13.9 % by weight of fibres are provided, with an indication of the split between soluble and insoluble fibres.

Moreover, in recent years, consumers have become more attentive to the label of food products, including cereal bars, and they expect manufactured food products to be as natural and healthy as possible. Therefore, it is desirable to develop food processing technologies that limit the use of food additives, even when such food additives have been fully cleared by health or food safety authorities. This will further help communicate a wholesome and natural image on the cereal bar.

Given the health benefits of whole grain cereal, it is also desirable to provide as much intact dietary fibres per serving as possible. Cereal bars are a good vehicle for delivering whole grain cereal. To increase the whole grain content of a serving, it is of course possible to increase the serving size. But this is not desirable as it results in a greater calories intake. Another difficulty in increasing the whole grain content is that it usually impacts the taste, texture and overall appearance of the cereal bar, as well as its processability.

The consumer is not willing to compromise on cereal bar organoleptic properties, in order to increase his daily whole grain intake. Taste, texture, overall appearance are such organoleptic properties.

Obviously, industrial line efficiency is a mandatory requirement in the food industry. This includes handling and processing of raw materials, forming of the cereal bar, packaging and later storing, in warehouses, on the shelf or at home.

Therefore, it is desirable to provide a process for preparing whole grain cereal bars that are rich in whole grain and in dietary fibres, while maintaining a low calorie intake, that provide an excellent consumption experience to the consumer, and that may be easily industrialised at a reasonable cost.

### SUMMARY OF THE INVENTION

To this end, the inventors have proposed to increase the whole grain content of cereal bars by using a binder made of enzymatically hydrolysed whole grain flour, wherein the dietary fibres from whole grain flour are preserved from hydrolysis. Only the proteins and the starch of the whole grain flour are hydrolysed, to provide a semi-liquid product with a reduced viscosity compared to the starting material making easier the overall processing.

Indeed, for a given serving size, the maximum amount of naturally occurring dietary fibres is limited by the amount of whole grain cereal pieces in the cereal bar. A balance must be struck between the cereal pieces and the binder in the cereal bar, knowing that parameters such as respective water activities of the cereal pieces and the binder, and total solids content of the binder have a strong impact on processability of the mix of cereal pieces and binder, on the shelf-life and on the structure or texture of the cereal bar.

To this end, the inventors have developed a process for preparing a cereal bar comprising the steps of i) blending a food pieces mix comprising whole grain cereal pieces, with a whole grain cereal binder, and ii) forming a cereal bar from said mixture. The binder is obtainable by a process comprising the steps of:
a) contacting a whole-grain cereal flour in water with an enzyme composition comprising at least a protease and an alpha-amylase, both showing no hydrolytic activity towards dietary fibres, to provide a whole-grain flour hydrolysate,
b) inactivating said enzymes when said hydrolysate has reached a stable viscosity comprised between 100 and 30,000 mPa.s measured at 65°C and a total solid content of 25 to 50% by weight,
c) concentrating and mixing with a humectant and a fat component, to provide a whole-grain binder.

A cereal bar obtainable by this process is also part of the invention, as well as a process for preparing a whole-grain cereal binder comprising steps a) b) and c) detailed above.

These and other aspects, features and advantages of the invention will become apparent to those skilled in the art upon reading the disclosure provided here in connection with the attached drawings. The detailed description, while indicating preferred embodiments of the invention, is only given by way of illustration.

### BRIEF DESCRIPTION OF THE DRAWINGS

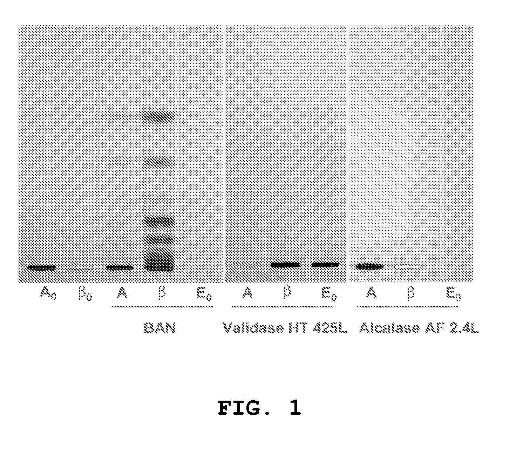

Figure 1 shows a thin layer chromatography analysis of various enzymes put in contact with dietary fibres. The legend for the different tracks is the following:
   A₀: pure arabinoxylan spot (blank)
   β₀: pure beta-glucan spot (blank)
   A: arabinoxylan spot after incubation with the enzyme noted below the track (BAN, Validase HT 425L and Alcalase AF 2.4L)
   β: beta-glucan spot after incubation with the enzyme noted below the track (BAN, Validase HT 425L and Alcalase AF 2.4L)
   E₀ : enzyme spot (blank)

### DETAILED DESCRIPTION OF THE INVENTION

It must be noted that in this specification and the appended claims, the singular forms "a", "an", and "the" include plural referents unless the context clearly dictates otherwise. Unless defined otherwise, all technical and scientific terms have the same meaning as commonly understood by one of ordinary skill in the art to which this invention belongs.

Cereals are monocotyledonous plants of the Poaceae family (grass family) cultivated for their edible, starchy grains. Examples of cereals include barley, brown rice, wild rice, buckwheat, bulgur, corn, millet, oat, sorghum, spelt, triticale, rye, wheat, wheat berries, teff, canary grass, Job's tears and fonio. Plant species that do not belong to the grass family also produce starchy seeds or fruits that may be used in the same way as cereal grains, are called pseudocereals. Examples of pseudocereals include amaranth, buckwheat, tartar buckwheat and quinoa. When designating cereals, this will include both cereal and pseudocereals.

Whole grain cereal products are products made from unrefined cereal grains. Whole grains contain the entire edible parts of a grain: germ, endosperm and bran. Whole grain products may be provided in a variety of forms such as ground, flaked, cracked or other forms, as is commonly known in the milling industry.

Flour and especially whole grain flour is the product resulting of grain milling. Flour can be heat treated to limit rancidity and microbial count. In the context of the invention, unrefined whole grain flour is used.

Dietary fibres are the edible parts of plants that are not broken down by digestion enzymes. Dietary fibres are fermented in the human large intestine by the microflora. There are two types of fibres: soluble fibres and insoluble fibres. Both soluble and insoluble dietary fibres can promote a number of positive physiological effects, including a good transit through the intestinal tract which helps to prevent constipation, or a feeling of fullness. Health authorities recommend a consumption of between 20 and 35 g per day of fibres, depending on the weight, gender, age and energy intake.

Soluble fibres are dietary fibres that undergo complete or partial fermentation in the large intestine. Examples of soluble fibres from cereals include beta-glucans, arabinoxylans, arabinogalactans and resistant starch type 2 and 3, and oligosaccharides deriving from the latter. Solube fibres from other sources include pectins, acacia gum, gums, alginate, agar, polydextrose, inulins and galacto-oligosaccharides for instance. Some soluble fibres are called prebiotics, because they are a source of energy for the probiotics present in the large intestine. Further benefits of soluble fibres include blood sugar control, which is important in diabetes prevention, control of cholesterol, or risk reduction of cardiovascular disease.

Insoluble fibres are the dietary fibres that are not fermented in the large intestine or only slowly digested by the intestinal microflora. Examples of insoluble fibres include celluloses, hemicelluloses, resistant starch type 1 and lignins. Further benefits of insoluble fibres include promotion of the bowel function through stimulation of the peristalsis, which causes the muscles of the colon to work more, become stronger and function better. There is also evidence that consumption of insoluble fibres may be linked to a reduced risk of gut cancer.

As detailed above, the process for preparing a cereal bar comprises the following two steps: i) blending a food pieces mix with a whole grain cereal binder, and ii) forming a cereal bar from said mixture.

The binder is heated to a flowable state, preferably at a temperature from 65°C to 85°C. Usually, the temperature is comprised between 75°C and 80°C. The optimal temperature can be determined by a skilled artisan. It is intimately and gently blended with a food pieces mix. Usually, the food pieces are dry pieces. A single-screw mixer or a batch cooker may be used for heating the binder and blending it with the food pieces mix. The binder should be fluid enough to coat the food pieces evenly. At a lower temperature, the binder remains too viscous and there is a risk that dry food pieces get broken by friction. A higher temperature, does not bring an improved blending, may provoke undesired reactions in the binder and is associated to increased energy consumption.

The main component of the food pieces mix is whole grain cereal pieces, preferably dry pieces. Whole grain cereal pieces are food pieces made from or with whole grain cereals. Whole grain cereal pieces can be 100% whole grain products, but this is not systematic and they can be made partly from whole grain cereal and from refined cereal products. Whole grain cereal pieces can contain various secondary ingredients such as sweeteners or flavours. Various technologies exist for making such pieces, like extrusion, flaking or batch-cooking.

In granola bars, the food pieces mix can also comprise optional components such as dried fruit pieces, nut pieces, and chocolate pieces. Mixtures thereof are also possible. Dried fruits may be orange, strawberry, grape, apple, pear, banana, apricot. Nuts may be chestnut, walnut, peanuts, almonds, hazelnuts.

In savoury bars, the food pieces mix can comprise fish pieces, meat pieces, cheese pieces, vegetable pieces, and mixtures thereof.

The preparation of the whole grain cereal binder will be described below.

The next step is the forming of cereal bars. In an embodiment, the final mixture is formed into a layer by feeding the mixture between two rollers. The layer is then cooled down. Cooling the layer of mixture may be achieved in a cooling tunnel. The final temperature is chosen so that the binder sets. The binder viscosity should be high enough to set rapidly the layer, still remain elastic to allow shaping and cutting. If the binder viscosity is too low, it will flow to the bottom of the layer, making the layer sticky and not cohesive. Usually, the final temperature is around 20°C. Then the layer is cut into bars. Former/previous methods are known in the art.

The binder is obtainable as follows. Whole grain cereal flour is mixed with water and contacted with an enzyme composition. The latter comprises at least a protease and an alpha-amylase, both showing no hydrolytic activity towards dietary fibres. The resulting composition is a whole grain flour hydrolysate.

The cereals are those mentioned above. Preferably, wheat, oat, barley, rice, sorghum and mixtures thereof are used. The whole grain cereal flour may be a mixture of different cereal flours or a flour of a single cereal. When it is desired to prepare a binder from different whole grain cereal, it is possible to prepare different cereal hydrolysates and then mix them according to the desired ratios, or to prepare a mix of different cereal flours and then prepare the hydrolysate directly from the cereal flours mix.

Proteases are enzymes allowing the hydrolysis of proteins. They are used to decrease the viscosity of the whole grain binder. Alcalase® 2.4L (EC 3.4.21.62), from Novozymes, Protease S from Amano are some examples.

Amylase (EC 3. 2. 1. 1) is an enzyme classified as a saccharidase: an enzyme that cleaves polysaccharides. It is mainly a constituent of pancreatic juice and saliva, needed for the breakdown of long-chain carbohydrates such as starch, into smaller units. Here, alpha-amylase is used to hydrolyse gelatinized starch in order to decrease the viscosity of the whole grain binder. Validase HT 425L from Valley Research, Fungamyl from Novozymes are examples of alpha-amylase. Those enzymes show no activity towards the dietary fibres. On the contrary, BAN from Novozymes degrades some dietary fibres besides starch.

From a thin layer chromatography analysis (figure 1), it has been shown that the selected alpha-amylase and protease have no hydrolytic activity on beta-glucan and arabinoxylan, while BAN, another commercial alpha-amylase preparation, does hydrolyse the fibres.

The enzyme composition may further comprise an amyloglucosidase, preferably an amyloglucosidase that shows no hydrolytic activity towards dietary fibres.

Amyloglucosidase (EC 3.2.1.3) is an enzyme able to release glucose residues from starch, maltodextrins and maltose by hydrolysing glucose units from the non-reduced end of the polysaccharide chain. The sweetness of the preparation increases with the increasing concentration of released glucose.

Mixing may be performed in a double jacket cooker, though other industrial equipment may be used. A scraping mixer works continuously and scraps the inner surface of the mixer. It avoids product burning and helps maintaining a homogeneous temperature. Thus enzyme activity is better controlled. Steam may be injected in the double jacket to increase temperature while cold water is used to decrease it.

In an embodiment, the enzyme composition and water are mixed together at room temperature, between 10 and 25°C. At this low temperature, the enzymes of the enzyme composition have a very weak activity. The whole grain flour is then added and the ingredients are mixed for a short period of time, usually less than 20 minutes, until the mixture is homogeneous.

The mixture is heated progressively or by thresholds to activate the enzymes and hydrolyse the components of the whole grain flour.

Hydrolysis results in a reduction of the viscosity of the mixture. When the hydrolysate has reached a stable viscosity comprised between 100 and 30,000 mPa.s measured at 65°C and a total solid content of 25 to 50% by weight, the enzymes are inactivated by heating the hydrolysate at a temperature above 100°C, preferably by steam injection at 120°C.

Finally, the whole grain binder is prepared by concentrating the hydrolysate and mixing it with a humectant and a fat component. It is also possible to first mix the humectant and fat component into the hydrolysate, and then concentrate the mixture. This step is best performed in a double jacket cooker equipped with a vacuum device. Thus, evaporation temperature is lower than under atmospheric pressure.

Examples of humectants include glycerol and sorbitol. Fat components are preferably vegetable fats such as cocoa butter, rapeseed oil, sunflower oil or palm oil, preferably not hydrogenated. Other ingredients may be added to the hydrolysate, before or after concentration, before together with or after addition of the humectant and fat component. Preferably, the binder also contains sugar (such as sucrose crystalline, glucose syrup, inverted syrup, honey, malt extract or caster sugar). Supplementary ingredients of the binder include salt, vitamin and minerals, flavours, preservatives such as tocopherol, and emulsifiers, such as lecithin, protein powders, cocoa solid and other active ingredients, such as DHA, caffeine, prebiotic, LcPUFA....

### [COMPLETER AU BESOIN]

To improve the shelf life of the cereal bar, it is important that water activity (Aw) and moisture are monitored. The overall Aw of a cereal bar must be below 0.6 in order to prevent microbial growth and food spoilage (moulds and yeast). Final bar Aw is adjusted by lowering binder moisture through evaporation and by use of humectant. By lowering binder aW from 0.55 to 0.35, the bar becomes less chewy and crispier, to harder. A too high moisture content will make the bar sticky, very deformable and chewy. A too low moisture content will make the bar crispy, hard, difficult to form evenly.

In the binder, the final Aw is between 0.40 and 0.50, preferably from 0.45 to 0.47, with a total solids content from 87% to 90%, preferably from 87% to 89%. The binder should show cohesion, but limited stickiness. When rolled between palms, it should come together and forms a ball, not getting spread onto palms.

The cereal bar preferably has an Aw of 0.35 +/-0.03.

Different parameters are mentioned in this specification. The methods for measuring them is explained below:
Water activity is measured according to the AOAC method 978.18 and performed at 25°C, after equilibrium is reached, using a HygroLab instrument from Rotronic.

Moisture content of the binder is determined by a direct Karl Fischer titration based on the ISO 5381:1983 method with the following adaptations:
The titration was performed at 50°C with a 751 GPD Titrino instrument from Metrohm. The test portion was added to a methanol/formamide mixture (2/1 v/v). After homogenization of the sample with a Polytron high speed homogenizer, the titration of the reacting water was performed with a Karl Fischer reagent (Hydranal® Composite 5, Sigma-Aldrich) up to the equivalence point, which was determined by voltametry.

Total solid content (%) of the binder equals 100 minus moisture content (%) of the binder as measured by the adapted Karl Fischer titration described above.

The total solid content of the hydrolysate was determined using a Moplant equipment for rapid total solids determination. The test portion was weighed and evaporated on a hotplate at 130°C. After evaporation of the liquid, the sample was further dried in the vacuum oven at 130°C during 10 minutes, cooled in the cooling room during 2.5 minutes and the residue was weight.

Viscosity is measured using a Rapid Visco Analyser from Newport Scientific. The Rapid Visco Analyser measures the resistance of the product to the stirring action of a paddle. The viscosity was measured after 10 minutes stirring, at 65°C and 50 rpm.

As mentioned above, the invention also relates to a whole grain cereal bar obtainable by the process described herein. Preferably, the cereal bar comprises: from 40 to 60% by weight of whole grain cereal pieces, and from 30 to 60% by weight of a whole grain cereal binder containing at least 2% by weight of dietary fibres. In an embodiment, the cereal bar contains at least 8 grams of whole grain per serving. Usually, a serving is between 20 g and 60 g. In another embodiment, the cereal bar comprises at least 30% by weight of whole grain. Depending on the whole grain content of the whole grain cereal pieces and of the whole grain binder, it may be possible to reach a whole grain content of 60% in the cereal bar (example 3).

An interest of the invention, and particularly of the process for preparing the whole grain binder, is that it allows reducing the sugar content of the binder when compared to products on the market. When an amyloglucosidase is used in the enzyme composition, it may become possible to dispense with sugar. In an embodiment, the whole grain binder comprises less than 50% by weight of sucrose. In an embodiment, the whole grain binder contains between 15 and 30% by weight of sucrose. In another embodiment, the binder comprises less than 10% by weight of sucrose. In another embodiment, the binder comprises 30 to 60% by weight of whole grain and from 2 to 6% by weight of fibres from whole grain.

### EXAMPLES

### Example 1: Preparation of a hydrolyzed whole grain

Enzymes are dosed according to the quantity of flour. Quantities of enzymes are different depending on the type of flour, as protein rates are different. The ratio water/flour can be adapted according to required moisture for the final liquid whole grain. Usually, the water/flour ratio is 60/40. Percents are by weight.

| Hydrolysed whole wheat | |
|---|---|
| Whole wheat flour | substrate |
| Enzyme amylase | 0.10% based on the substrate |
| Enzyme protease | 0.05% based on the substrate |
| | |

| Hydrolysed whole barley | |
|---|---|
| Whole barley flour | substrate |
| Enzyme amylase | 0.10% based on the substrate |
| Enzyme protease | 0.05% based on the substrate |
| | |

| Hydrolysed whole oat | |
|---|---|
| Whole oat flour | substrate |
| Enzyme amylase | 0.10% based on the substrate |
| Enzyme protease | 0.05% based on the substrate |
| | |

| Hydrolysed whole rice | |
|---|---|
| Whole rice flour | Substrate |
| Enzyme amylase | 0.10% based on the substrate |
| Enzyme protease | 0.01% based on the substrate |

The enzymes are mixed with water at room temperature (20°C) in a double jacket cooker. The flour is added, then mixed for 5 minutes until the mixture is homogeneous. The mixture is then heated to 50°C for the protease reaction, and maintained for 30 minutes, then heated again to 85°C and maintained for 10 min for the amylase reaction. Finally, the hydrolysed cereal is heated by steam injection to 120°C for 10 minutes, to inactivate the enzymes.

### Example 2: Preparation of a cereal bar containing 8g of whole grain

In the following examples, the hydrolyzed whole grain of example 1 is mixed with humectants, sugar, salt, vegetable fat, vitamins and minerals, flavours, preservatives, and emulsifiers, in a double jacket cooker equipped with a vacuum device. Mixing is performed while increasing the batch temperature up to 82°C under 300 mPa vacuum. This allows for evaporation of water and preparation of the binder.

The final Aw of the binder is 0.4 +/-0.05, with a total solids content of about 87% to 89%. The binder shows cohesion, but limited stickiness. When rolled between palms, it should come together and forms a ball, not getting spread onto palms.

### Whole grain cereal bar with chocolate pieces

| Ingredient | % by weight |
|---|---|
| Whole grain cereal flakes | 40.3% |
| Chocolate foot | 10% |
| Chocolate pieces | 5% |
| Sugar | 18.4% |
| Humectants (glycerol) | 3.8% |
| Vegetable fat | 2.3% |
| Vitamins & minerals | 1.4% |
| Flavour | 0.2% |
| Emulsifier & Preservatives | 0.2% |
| Hydrolysed whole barley TS 35 | 9.5% |
| Hydrolysed whole wheat TS 40 | 10.2% |
| Total input ingredients | 100.000 |

In this bar, the binder represents 46% by weight of the bar and it contains 5.3% by weight whole grain fibres. The cereal bar contain 43.88% by weight of whole grain.

### Whole grain cereal bar with chocolate pieces

| Ingredient | % by weight |
|---|---|
| Flakes | 34.00% |
| Flakes 100% whole grain | 7.00% |
| Chocolate foot | 10% |
| Chocolate pieces | 5% |
| Sugar | 20.00% |
| Humectants | 8.00% |
| Vegetable fat | 2.30% |
| Vitamins & minerals | 1.40% |
| Flavour | 0.20% |
| Emulsifier & Preservatives | 0.20% |
| Preservatives | 0.00% |
| Hydrolysed whole wheat TS 40 | 11.00% |
| Total input ingredients | 100.00% |

In this bar, the binder represents 44% by weight of the bar and it contains 2,16% by weight whole grain fibres. The cereal bar contain 18% by weight of whole grain.

### Whole grain cereal bar with orange pieces

| Ingredient | % by weight |
|---|---|
| Flakes 100% whole grain | 23.00 |
| Orange pieces | 5.70 |
| Extruded crisps | 12.32 |
| Chocolate foot | 10.00 |
| Honey | 6.00 |
| Humectants | 6.00 |
| Vegetable fat | 3.30 |
| Flavour | 0.22 |
| Emulsifier & Preservatives | 0.08 |
| Hydrolysed whole wheat TS 55 | 34.11 |
| Total input ingredients | 100 |

In this bar, the binder represents 54.68% by weight of the bar and it contains 6.85% by weight whole grain fibres. The cereal bar contain 57.11% by weight of whole grain.

Although preferred embodiments have been described in the foregoing description, it will be understood that the invention is not limited to the specific embodiments disclosed. Various modifications may become apparent to those of ordinary skill in the art and may be acquired from practice of the invention. It will be understood that the materials used may be slightly different or modified from the descriptions herein without departing from the methods and compositions disclosed and taught by the present invention.

## Claims

1. A process for preparing a cereal bar comprising the steps of i) blending a food pieces mix comprising whole grain cereal pieces, with a whole grain cereal binder, and ii) forming a cereal bar from said mixture,
wherein said binder is obtainable by a process comprising the steps of:
a) contacting a whole grain cereal flour in water with an enzyme composition comprising at least a protease and an alpha-amylase, both showing no hydrolytic activity towards dietary fibres, to provide a whole grain flour hydrolysate,
b) inactivating said enzymes when said hydrolysate has reached a stable viscosity comprised between 100 and 30,000 mPa.s measured at 65°C and a total solid content of 25 to 50% by weight,
c) concentrating and mixing with a humectant and a fat component, to provide a whole grain binder.

2. The process according to claim 1, wherein said enzyme composition further comprises an amyloglucosidase.

3. The process according to claim 2, wherein said amyloglucosidase shows no hydrolytic activity towards dietary fibres.

4. The process according to any one of claims 1 to 3, wherein the cereal bar comprises:
- from 40 to 60% by weight of whole grain cereal pieces,
- from 30 to 60% by weight of a whole grain cereal binder containing at least 2% by weight of dietary fibres,
wherein the cereal bar contains at least 8 grams of whole grain per serving.

5. The process according to any one of claims 1 to 4, wherein said binder comprises less than 50% by weight of sucrose.

6. The process according to any one of claims 1 to 5, wherein the cereal bar comprises at least 30% by weight of whole grain.

7. The process according to any one of claims 1 to 6, wherein the binder comprises 30 to 60% by weight of whole grain and from 2 to 6% by weight of fibres from whole grain.

8. The process according to any one of claims 1 to 7, wherein the food pieces mix further comprises pieces selected from dried fruit pieces, nut pieces, chocolate pieces, fish pieces, meat pieces, cheese pieces and vegetable pieces.

9. The process according to any of the preceding claims, wherein the whole grain cereal is selected from the group consisting of barley, brown rice, wild rice, buckwheat, bulgur, corn, millet, oat, quinoa, sorghum, spelt, triticale, rye, wheat, wheat berries, teff, canary grass, Job's tears, fonio, amaranth, buckwheat, tartar buckwheat, quinoa wild rice, and mixtures thereof.

10. A cereal bar obtainable by a process according to any one of claims 1 to 9.

11. A cereal bar according to claim 10, comprising:
- from 40 to 60% by weight of whole grain cereal pieces,
- from 30 to 60% by weight of a whole grain cereal binder containing at least 2% by weight of dietary fibres,
wherein the cereal bar contains at least 8 grams of whole grain per serving.

12. A cereal bar according to claim 10 or 11, comprising at least 30% by weight of whole grain.

13. A cereal bar according to any one of claims 10 to 12, wherein the binder comprises 30 to 60% by weight of whole grain and from 2 to 6% by weight of fibres from whole grain.

14. A process for preparing a whole grain cereal binder comprising the steps of:
a) contacting a whole grain cereal flour in water with an enzyme composition comprising at least a protease and an alpha-amylase having no hydrolytic activity towards dietary fibres, to provide a whole grain flour hydrolysate,
b) inactivating said enzymes when said hydrolysate has reached a stable viscosity comprised between 100 and 30,000 mPa.s measured at 65°C and a total solid content of 25 to 50% by weight,
c) concentrating and mixing with a humectant and a fat component, to provide a whole grain binder.
